# EUROPEAN PATENT SPECIFICATION

(11) **EP 3 787 712 B1**
(45) Date of publication and mention of the grant of the patent: **20.12.2023**
(21) Application number: 19724040.1
(22) Date of filing: 02.05.2019
(51) Int. Cl.: A61M 5/142, A61M 5/158

(54) **MEDICAL DEVICE WITH EXTENDED WEAR ADHESIVE PATCH**
MEDZINIZSCHES GERAET MIT LAENGER TRAGBAREM KLEBEPATCH
DISPOSITIF MÉDICAL AVEC PATCH ADHÉSIF D'UTILISATION PROLONGÉE

(30) Priority: 04.05.2018 US 201862667349 P; 01.05.2019 US 201916400878
(43) Date of publication of application: 10.03.2021
(73) Proprietor: Medtronic MiniMed, Inc., Northridge, CA 91325-1219 (US)
(72) Inventor: DANG, Kiem, Thousand Oaks, California 91360 (US); CHATTARAJ, Sarnath, Simi Valley, California 93063 (US); FUSSELMAN, Hsi C., Simi Valley, California 93065 (US); ZHANG, Guangping, Calabasas, California 91302 (US)
(74) Representative: Schröer, Gernot H.
(86) International application number: PCT/US2019/030434
(87) International publication number: WO 2019/213419

(56) References cited:
- EP-A2- 0 573 137
- US-A1- 2004 116 866
- US-A1- 2005 113 761
- US-A1- 2016 213 322
- US-B2- 9 254 373

## Description

### FIELD

The subject matter described herein relate generally to medical devices, such as those which are coupled to an anatomy with an adhesive patch. More particularly, the subject matter relates to a medical device that has an adhesive patch that provides for extended wear.

### BACKGROUND

Certain diseases or conditions may be treated, according to modem medical techniques, by delivering a medication or other substance to the body of a user, either in a continuous manner or at particular times or time intervals within an overall time period. For example, diabetes is commonly treated by delivering defined amounts of insulin to the user at appropriate times. Some common modes of providing insulin therapy to a user include delivery of insulin through manually operated syringes and insulin pens. Other modem systems employ programmable fluid infusion devices (e.g., insulin pumps) to deliver controlled amounts of insulin to a user. In certain instances, these fluid infusion devices require an insertion set, such as an infusion set, to be coupled to the body of a user for the delivery of the insulin. Generally, the infusion set is coupled to the fluid infusion device via hollow tubing, which provides a fluid flow path from the fluid infusion device to the user. The infusion set requires a portion of a cannula, for example, to be inserted under the skin of the user to deliver the controlled amounts of insulin from the fluid infusion device to the user via the infusion set while the infusion set is coupled to the body of the user.

Typically, the infusion set is coupled to the body of the user with an adhesive patch. In certain instances, due to environmental factors, such as water encountered during bathing, swimming, etc., for example, the adhesive patch may peel off the skin of the user and result in an uncoupling of the adhesive patch from the body of the user. The uncoupling of the adhesive patch from the user reduces a wear life of the infusion set, which is inconvenient to a user. Further, other medical devices, such as continuous glucose sensors or monitors, patch-based fluid infusion systems, etc., which employ an adhesive patch to couple the medical device to an anatomy may also encounter instances where the adhesive patch may peel off the skin of the user due to environmental factors, such as water encountered while bathing, swimming, etc., for example. US2016/021332 discloses a system for mounting a device on a user having a flexible mounting layer adhered on one face to a rigid mounting platform and on the other to the body of the user. US9254373 likewise discloses an adhesive mount for placing a medical device on a user. US2005/0113761 discloses an infusion system having a delivery site adhered to the skin of a user. US2004/0116866 describes a skin attachment apparatus having a carrier layer to be adhered to the skin. EP0573137 shows a multilayer patch for adhering an oxygen sensor to a user having a layer containing a perfusion enhancer a layer for regulating the flux into the skin.

Accordingly, it is desirable to provide a medical device, such as an infusion set, continuous glucose sensors or monitors, patch-based fluid infusion systems, etc., with an extended wear adhesive patch, in which a peeling or uncoupling of the adhesive patch is inhibited even while encountering environmental factors, like water. Furthermore, other desirable features and characteristics will become apparent from the subsequent detailed description and the appended claims, taken in conjunction with the accompanying drawings and the foregoing technical field and background.

### SUMMARY

The invention is as set out in the attached claims. The techniques of this disclosure generally relate to a medical device, such as an infusion set, continuous glucose sensors or monitors, patch-based fluid infusion systems, etc. with an extended wear adhesive patch that resists environmental factors while maintaining user comfort.

Provided is an adhesive patch for a medical device. The adhesive patch includes a first backing layer to be coupled to the medical device. The first backing layer is composed of a first material. The adhesive patch includes a first adhesive layer coupled to the first backing layer and a second backing layer coupled to the first adhesive layer. The second backing layer is composed of a second material, and the second material is different than the first material. The adhesive patch includes a biocompatible second adhesive layer coupled to the second backing layer, and the second adhesive layer is to be coupled to an anatomy.

Further provided is a medical device. The medical device includes a hub and an adhesive patch coupled to the hub. The adhesive patch is to couple the hub to an anatomy. The adhesive patch includes a first backing layer coupled to the medical device. The first backing layer is composed of a first material. The adhesive patch includes a first adhesive layer coupled to the first backing layer and a flexible second backing layer coupled to the first adhesive layer. The second backing layer is composed of a second material that is different than the first material. The adhesive patch
includes a biocompatible second adhesive layer coupled to the second backing layer. The second adhesive layer is to be coupled to the anatomy. The adhesive patch includes a liner removably coupled to the second adhesive layer.

Also provided is an infusion unit. The infusion unit includes a hub to define a fluid flow path to receive a fluid and an adhesive patch coupled to the hub. The adhesive patch is to couple the hub to an anatomy. The adhesive patch includes a first backing layer coupled to the hub via ultrasonic welding. The first backing layer is composed of a first material that facilitates the ultrasonic welding of the first backing layer to the hub. The adhesive patch includes a first adhesive layer coupled to the first backing layer and a flexible, water resistant second backing layer coupled to the first adhesive layer. The second backing layer is composed of a second material that is different than the first material. The adhesive layer includes a biocompatible second adhesive layer coupled to the second backing layer. The second adhesive layer is to be coupled to the anatomy. The adhesive patch includes a liner removably coupled to the second adhesive layer.

This summary is provided to introduce a selection of concepts in a simplified form that are further described below in the detailed description. The details of one or more aspects of the disclosure are set forth in the accompanying drawings and the description below. Other features, objects, and advantages of the techniques described in this disclosure will be apparent from the description and drawings, and from the claims. This summary is not intended to identify key features or essential features of the claimed subject matter, nor is it intended to be used as an aid in determining the scope of the claimed subject matter.

### BRIEF DESCRIPTION OF DRAWINGS

A more complete understanding of the subject matter may be derived by referring to the detailed description and claims when considered in conjunction with the following figures, wherein like reference numbers refer to similar elements throughout the figures.
FIG. 1 is a perspective view of an exemplary fluid infusion device including a medical device, such as an infusion set, with an extended wear adhesive patch in accordance with various embodiments;
FIG. 2 is a perspective view of the infusion set of FIG. 1, which includes the extended wear adhesive patch in accordance with various embodiments;
FIG. 3 is a cross-sectional view of the extended wear adhesive patch of the infusion set of FIG. 2, taken along line 3-3 of FIG. 2;
FIG. 4 is a schematic illustration of a method for assembling the extended wear adhesive patch in accordance with various embodiments;
FIG. 5 is a perspective schematic view of a medical device, such as a physiological characteristic sensor, with the extended wear adhesive patch of FIG. 1 in accordance with various embodiments;
FIG. 6 is a perspective schematic view of a medical device, such as a fluid infusion system, with the extended wear adhesive patch of FIG. 1 in accordance with various embodiments;
FIG. 7 is a cross-sectional view of an exemplary extended wear adhesive patch for use with the infusion set of FIG. 2, the physiological characteristic sensor of FIG. 5 and/or the fluid infusion system of FIG. 6, taken from the perspective of line 3-3 of FIG. 2;
FIG. 8 is a cross-sectional view of another exemplary extended wear adhesive patch for use with the infusion set of FIG. 2, the physiological characteristic sensor of FIG. 5 and/or the fluid infusion system of FIG. 6, taken from the perspective of line 3-3 of FIG. 2; and
FIG. 9 is a cross-sectional view of yet another exemplary extended wear adhesive patch for use with the infusion set of FIG. 2, the physiological characteristic sensor of FIG. 5 and/or the fluid infusion system of FIG. 6, taken from the perspective of line 3-3 of FIG. 2.

### DETAILED DESCRIPTION

The following detailed description is merely illustrative in nature and is not intended to limit the embodiments of the subject matter or the application and uses of such embodiments. As used herein, the word "exemplary" means "serving as an example, instance, or illustration." Any implementation described herein as exemplary is not necessarily to be construed as preferred or advantageous over other implementations. Furthermore, there is no intention to be bound by any expressed or implied theory presented in the preceding technical field, background, brief summary or the following detailed description.

Certain terminology may be used in the following description for the purpose of reference only, and thus are not intended to be limiting. For example, terms such as "top", "bottom", "upper", "lower", "above", and "below" could be used to refer to directions in the drawings to which reference is made. Terms such as "front", "back", "rear", "side", "outboard", and "inboard" could be used to describe the orientation and/or location of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second", and other such numerical terms referring to structures do not imply a sequence or order unless clearly indicated by the context.

As used herein, the term "axial" refers to a direction that is generally parallel to or coincident with an axis of rotation, axis of symmetry, or centerline of a component or components. For example, in a cylinder or disc with a centerline and generally circular ends or opposing faces, the "axial" direction may refer to the direction that generally extends in parallel to the centerline between the opposite ends or faces. In certain instances, the term "axial" may be utilized with respect to components that are not cylindrical (or otherwise radially symmetric). For example, the "axial" direction for a rectangular housing containing a rotating shaft may be viewed as a direction that is generally parallel to or coincident with the rotational axis of the shaft. Furthermore, the term "radially" as used herein may refer to a direction or a relationship of components with respect to a line extending outward from a shared centerline, axis, or similar reference, for example in a plane of a cylinder or disc that is perpendicular to the centerline or axis. In certain instances, components may be viewed as "radially" aligned even though one or both of the components may not be cylindrical (or otherwise radially symmetric). Furthermore, the terms "axial" and "radial" (and any derivatives) may encompass directional relationships that are other than precisely aligned with (e.g., oblique to) the true axial and radial dimensions, provided the relationship is predominately in the respective nominal axial or radial direction. As used herein, the term "transverse" denotes an axis that crosses another axis at an angle such that the axis and the other axis are neither substantially perpendicular nor substantially parallel.

The following description generally relates to a medical device, for example, an infusion set of the type used in treating a medical condition of a user. The infusion set infuses a fluid into a body of the user. The non-limiting examples described below relate to an infusion set used in the treatment of diabetes, although embodiments of the disclosed subject matter are not so limited. Accordingly, the infused medication fluid is insulin in certain embodiments. In alternative embodiments, however, many other fluids may be administered through infusion such as, but not limited to, disease treatments, drugs to treat pulmonary hypertension, iron chelation drugs, pain medications, anti-cancer treatments, medications, vitamins, hormones, or the like. In other embodiments, the medical device comprises continuous glucose sensors or monitors, patch-based fluid infusion systems, etc. Generally, the adhesive patch discussed herein may be employed with any suitable device that benefits from extended adhesive wear life, and thus, the use of the adhesive patch with a medical device is merely one example of a use of the present disclosure. For the sake of brevity, conventional features and characteristics related to infusion system operation, insulin pump and/or infusion set operation, fluid reservoirs, and fluid syringes may not be described in detail here. Examples of infusion pumps and/or related pump drive systems used to administer insulin and other medications may be of the type described in, but not limited to: U.S. Patent Publication Nos. 2009/0299290 and 2008/0269687; U.S. Patent Nos. 4,562,751; 4,678,408; 4,685,903; 5,080,653; 5,505,709; 5,097,122; 6,485,465; 6,554,798; 6,558,351; 6,659,980; 6,752,787; 6,817,990; 6,932,584; 7,621,893; 7,828,764; and 7,905,868.

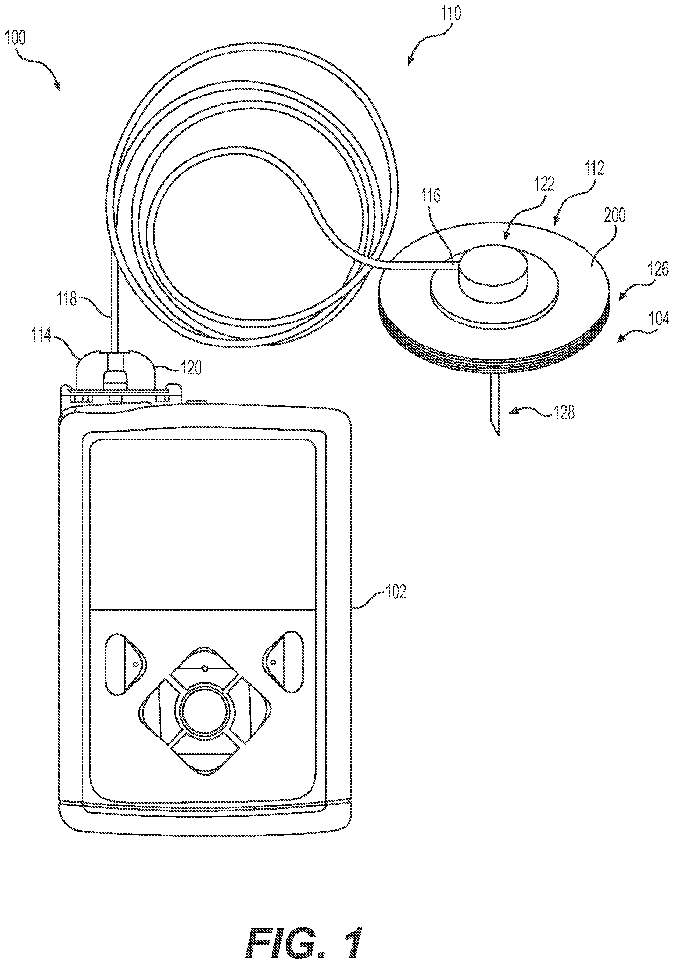

FIG. 1 is a perspective view of an exemplary embodiment of a fluid infusion system 100. The fluid infusion system 100 includes two main components: a fluid infusion device 102 (e.g., an insulin pump) and an infusion set 104, which is coupled to the fluid infusion device 102 as depicted in FIG. 1. In one example, the infusion set 104 includes, without limitation: a hollow fluid supply line or tube 110, a medical device or an infusion unit 112 and a connector assembly 114. The infusion unit 112 is coupled to a first end 116 of the tube 110 and the connector assembly 114 is coupled to a second end 118 of the tube 110. The fluid infusion device 102 is carried or worn by the user, and the infusion set 104 terminates at the infusion unit 112 to enable the fluid infusion device 102 to deliver fluid to the body of the user via the tube 110. Thus, the infusion unit 112 is coupled to the body of the user, as described in more detail below. The fluid infusion device 102 may leverage a number of conventional features, components, elements, and characteristics of existing fluid infusion devices. For example, the fluid infusion device 102 may incorporate some of the features, components, elements, and/or characteristics described in United States Patent Nos. 6,485,465 and 7,621,893. It should be noted that the fluid infusion device 102 illustrated herein is merely exemplary, as any suitable fluid infusion device can be employed with the infusion set 104.

The fluid infusion device 102 accommodates a fluid reservoir (hidden from view in FIG. 1) for the fluid to be delivered to the user. The tube 110 represents the fluid flow path that couples the fluid reservoir to the infusion unit 112. When installed as depicted in FIG. 1, the tube 110 extends from the fluid infusion device 102 to the infusion unit 112, which in turn provides a fluid pathway to the body of the user. For the illustrated embodiment, the connector assembly 114 is realized as a removable reservoir cap 120 (or fitting) that is suitably sized and configured to accommodate replacement of fluid reservoirs (which are typically disposable) as needed. In this regard, the reservoir cap 120 is designed to accommodate the fluid path from the fluid reservoir to the tube 110.

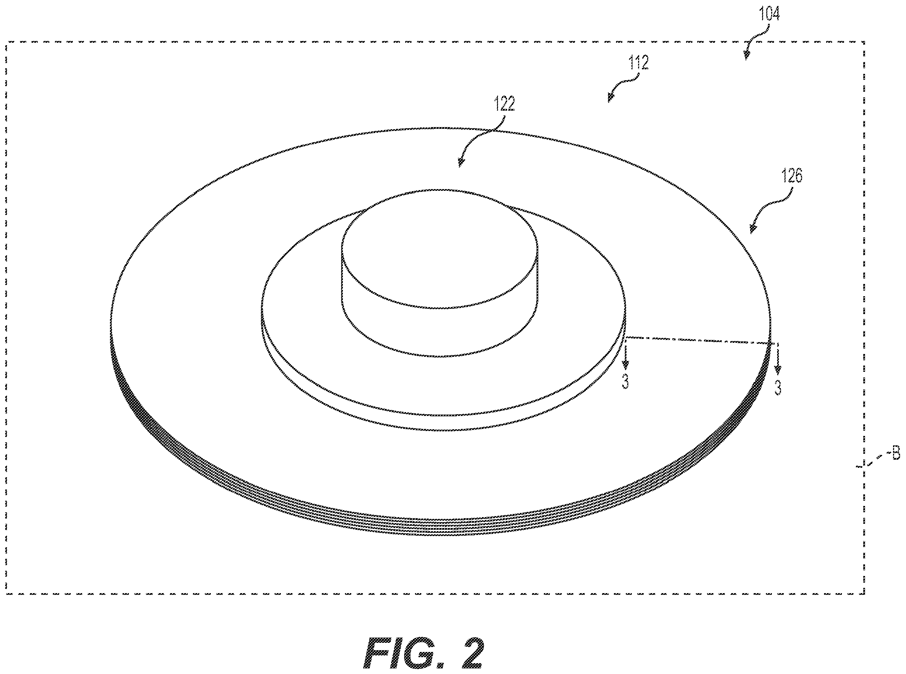

With reference to FIG. 2, the infusion unit 112 of the infusion set 104 is shown coupled to an anatomy or a body B of a user. In FIG. 2, the tube 110, including the first end 116, is removed for clarity. The infusion unit 112 delivers fluid from the fluid reservoir associated with the fluid infusion device 102 (FIG. 1) received through the tube 110 into the body of the user. The infusion unit 112 includes a hub 122, a coupling device or adhesive patch 126 and a fluid outlet 128 (FIG. 1). As shown in FIG. 2, the adhesive patch 126 removably couples or secures the hub 122 of the infusion unit 112 to the body B of the user. With reference back to FIG. 1, as the hub 122 and the fluid outlet 128 may leverage a number of conventional features, such as those of the MiniMed Quick-set^{®} infusion set commercially available from Medtronic MiniMed, Inc. of Northridge, California, the hub 122 and the fluid outlet 128 are not discussed in detail herein. Briefly, in this example, the hub 122 is annular, but the hub 122 may have any desired shape and configuration to facilitate fluid flow from the tube 110 through the infusion unit 112. The hub 122 couples the infusion unit 112 to the tube 110 and enables fluid flow from the tube 110 to exit through the fluid outlet 128. Thus, generally, the hub 122 includes one or more internal passageways that are coupled to the first end 116 of the tube 110 and enable fluid communication between the tube 110 and the fluid outlet 128. In one example, the fluid outlet 128 is cannulated, and a portion of the fluid outlet 128 is received into a portion of the body B (FIG. 2) of the user to enable the fluid from the fluid infusion device 102 to flow into the body B of the user. In one example, a proximal end of the fluid outlet 128 is received into the body B of the user (FIG. 2), and a distal end is fluidly coupled to the first end 116 of the tube 110. The proximal end includes a piercing tip; however, the proximal end may be atraumatic. Moreover, the fluid outlet 128 may be defined by the first end 116 of the tube 110 itself, if desired.

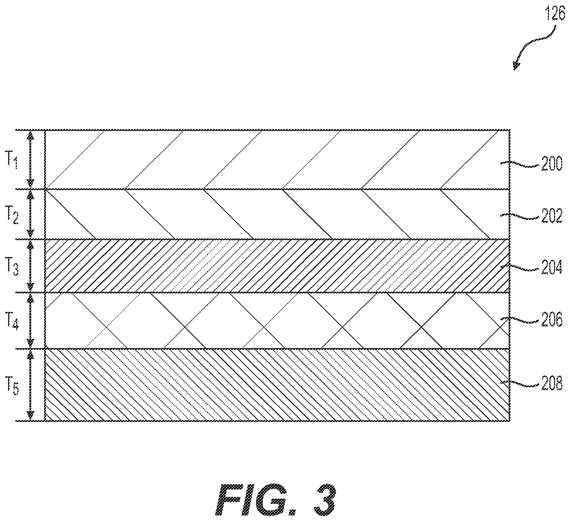

With reference to FIG. 3, the adhesive patch 126 is shown in greater detail. In one example, the adhesive patch 126 includes a first backing layer 200, a first adhesive layer 202, a second backing layer 204, a second adhesive layer 206 and a liner 208. Generally, the first backing layer 200 enables the coupling of the adhesive patch 126 to the hub 122 with more efficient manufacturing techniques, such as ultrasonic welding, while the second backing layer 204 provides flexibility, environmental factor resistance, such as water resistance, and breathability that provides user comfort. The combination of the first backing layer 200 with the second backing layer 204, thus, provides the adhesive patch 126 with longevity, environmental factor resistance, user comfort and ease of manufacturing. It should be noted that while the adhesive patch 126 is illustrated herein as being substantially circular in shape, the adhesive patch 126 may have any desired shape for the particular medical device, including, but not limited to non-circular, trilobular, etc.

The first backing layer 200 couples the adhesive patch 126 to the hub 122. In one example, the first backing layer 200 is composed of a thermoplastic woven or non-woven material, including, but not limited to polycarbonate, polycarbonate urethane, polyamide, nylon, polypropylene, polyethylene, cellulose acetate, polyacrylate, polyester, etc. In one example, the first backing layer 200 is composed of a thermoplastic woven or non-woven material that is suitable for ultrasonic welding to the hub 122. In this non-limiting example, the first backing layer 200 is composed of polyester. The first backing layer 200 may also be composed of a thermoplastic woven or non-woven material that has similar molecular structures as the hub 122. Generally, the first backing layer 200 provides ease of manufacturing of the medical device, such as the infusion unit 112 (FIG. 2) as the first backing layer 200 is able to be bonded to the hub 122 (FIG. 2) via ultrasonic welding. The first backing layer 200 has a thickness T1 of between about 3.0 mils to about 15 mils, and in one example, the thickness T1 is between about 8.0 mils to about 10 mils. The first backing layer 200 is physically coupled to or adhered to the first adhesive layer 202.

The first adhesive layer 202 couples the first backing layer 200 to the second backing layer 204. Stated another way, the first adhesive layer 202 is sandwiched between, and is adhesively coupled to the first backing layer 200 and the second backing layer 204. Thus, the first adhesive layer 202 is spaced apart from or not in direct contact with the body B (FIG. 2) or skin of the user. The first adhesive layer 202 may be coated onto or penetrated into the first backing layer 200. Thus, the first adhesive layer 202 and the first backing layer 200 may be separately or integrally formed. In one example, the first adhesive layer 202 is composed of a polyacrylate, silicone adhesive, epoxy, or an adhesive material that bonds securely to the second backing layer 204 without impacting the biocompatibility of the adhesive patch 126. The first adhesive layer 202 has a thickness T2 of between about 1.0 mils to about 5.0 mils, and in one example, the thickness T2 is between about 2.0 mils to about 4.0 mils.

The second backing layer 204 is coupled between the first adhesive layer 202 and the second adhesive layer 206. The second backing layer 204 is positioned between the first adhesive layer 202 and the second adhesive layer 206 (FIG. 2). In certain instances, when the second adhesive layer 206 (FIG. 2) penetrates through the second backing layer 204 or when under pressure, the second backing layer 204 may contact the skin of the user. In one example, the second backing layer 204 is composed of a stretchable, breathable and water-resistant biocompatible polymeric material, including, but not limited to polyethylene, polypropylene, silicone film/sheet, acrylic elastomer, polyurethane, etc. In this non-limiting example, the second backing layer 204 is composed of a polyurethane. Generally, the second backing layer 204 provides environmental factor resistance, such as water resistance, while maintaining breathability and flexibility, which provides the user with comfort during the extended use of the adhesive patch 126. Typically, due to the flexibility or stretchable nature of the second backing layer 204, the second backing layer 204 is not suited for coupling directly to the medical device, such as the hub 122 (FIG. 2). In this regard, the flexible or stretchable nature of the second backing layer 204 may require the use of double-sided adhesive tape to couple the second backing layer 204 to the medical device, such as the hub 122 (FIG. 2), which may be expensive, time consuming and generally difficult to manufacture. The second backing layer 204 has a thickness T3 of between about 3.0 mils to about 9.0 mils, and in one example, the thickness T3 is between about 5.0 mils to about 7.0 mils. Thus, generally, the second backing layer 204 has the thickness T3, which is less than the thickness T1 of the first backing layer 200. The second backing layer 204 is coupled to or adhered to the second adhesive layer 206.

The second adhesive layer 206 couples the second backing layer 204 to the liner 208. Stated another way, the second adhesive layer 206 is sandwiched between, and is adhesively coupled to the second backing layer 204 and the liner 208. The second adhesive layer 206 may be coated onto or penetrated into the second backing layer 204. Thus, the second adhesive layer 206 and the second backing layer 204 may be separately or integrally formed. As will be discussed, the liner 208 is removably coupled to the second adhesive layer 206, such that when the liner 208 is removed, the second adhesive layer 206 may be placed in direct contact with the body B (FIG. 2) or skin of the user. Thus, in this example, the second adhesive layer 206 couples the adhesive patch 126 to the body B (FIG. 2) of the user. In one example, the second adhesive layer 206 is composed of a biocompatible adhesive, including, but not limited to, polyacrylics, bio-based acrylate, ethylene-vinyl-acetate (EVA), and silicone adhesive. The second adhesive layer 206 has a thickness T4 of between about 1.0 mils to about 5.0 mils, and in one example, the thickness T4 is between about 2.0 mils to about 4.0 mils.

The liner 208 is removably coupled to the second adhesive layer 206. Generally, the liner 208 is coupled to at least a portion of the second adhesive layer 206, and is removable to facilitate coupling the adhesive patch 126 to the user. The liner 208 also protects the second adhesive layer 206. In one example, the liner 208 is composed of a silicone or wax coated paper, polyester, high-density polyethylene, etc. The liner 208 has a thickness T5 of between about 3.0 mils to about 9.0 mils, and in one example, the thickness T5 is between about 5.0 mils to about 7.0 mils.

Thus, in accordance with various embodiments, the first backing layer 200 is composed of a first material and the second backing layer 204 is composed of a second material that is different than the first material. The first adhesive layer 202 is composed of a third material, and the second adhesive layer 206 is composed of a fourth material that is different than the third material. In one example, each of the first material, the second material, the third material and the fourth material are different. In addition, in one example, one or more of the first backing layer 200, the first adhesive layer 202, the second backing layer 204, the second adhesive layer 206 and the liner 208 may have different sizes or shapes. For example, the liner 208 may be shaped to include a tab to enable the easy removal of the liner 208 from the second adhesive layer 206.

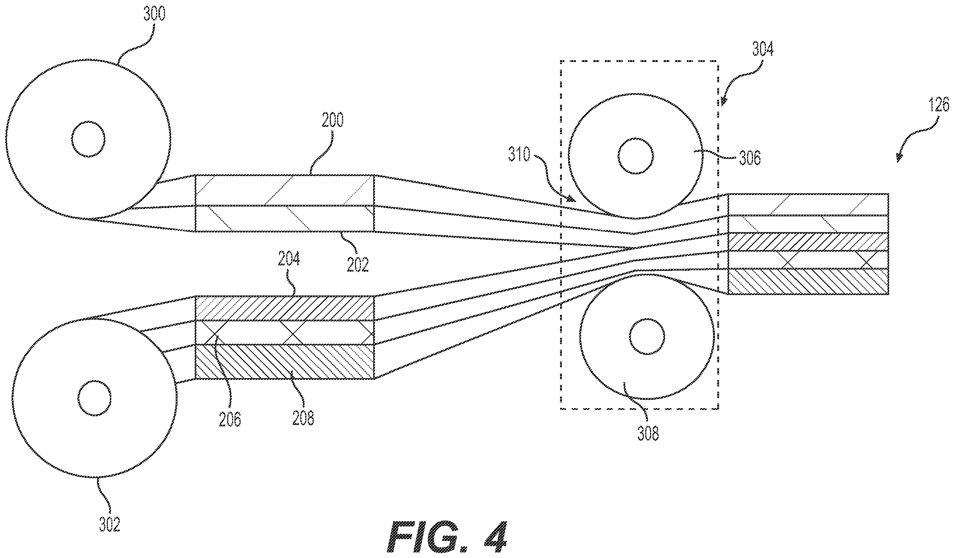

With reference to FIG. 4, an exemplary method of assembling the adhesive patch 126 is shown. In this example, the first backing layer 200 and the first adhesive layer 202 are provided as a first subassembly 300; and the second backing layer 204, the second adhesive layer 206 and the liner 208 are provided as a second subassembly 302. The first adhesive layer 202 may be coated on or penetrated into the first backing layer 200 in an initial processing step to form the first subassembly 300. The first subassembly 300 is illustrated herein as comprising a roll, however, the first subassembly 300 may be provided in any desirable form. The second adhesive layer 206 may be coated on or penetrated into the second backing layer 204 in an initial processing step, and the liner 208 may be adhered to the second adhesive layer 206 to form the second subassembly 302. The second subassembly 302 is illustrated herein as comprising a roll, however, the second subassembly 302 may be provided in any desirable form. The first subassembly 300 and the second subassembly 304 may be supported on respective pins or rods, which may be rotatable to advance the first subassembly 302 and the second subassembly 304 to a laminator 304.

The laminator 304 comprises a suitable laminator for coupling the first adhesive layer 202 of the first subassembly 300 to the second backing layer 204 of the second subassembly 302, including, but not limited to a heated roll laminator. In the example of the laminator 304 as a heated roll laminator, as is generally known, the laminator 304 includes two rollers 306, 308. One or both of the rollers 306, 308 may be heated to soften or melt at least the first adhesive layer 202 to couple or adhere the first adhesive layer 202 to the second backing layer 204 to form the adhesive patch 126. In certain embodiments, the rollers 306, 308 may be positioned to apply pressure to the first subassembly 300 and the second assembly 302 as the first subassembly 300 and the second assembly 302 passes between an opening 310 defined between the two rollers 306, 308 to aid in coupling the first adhesive layer 202 to the second backing layer 204. The rollers 306, 308 may be composed out of any suitable material, and may be mounted so as to be rotatable to advance the first subassembly 300 and the second assembly 302 through the opening 310. In certain embodiments, one or more of the rollers 306, 308 may be driven, via an output shaft of a motor, etc. to advance the first subassembly 300 and the second assembly 302 through the opening 310 to form the adhesive patch 126, which may be cut or stamped in a post processing step to arrive at the desired size and shape for use with the particular medical device. It should be noted that the use of a heated roll laminator is merely an example, as any suitable technique may be used to couple the first adhesive layer 202 of the first subassembly 300 to the second backing layer 204 of the second subassembly 302, including, but not limited to, pressure, light or ultrasonic welding. In addition, it should be noted that the method of assembling the adhesive patch 126 is merely exemplary as any suitable approach may be employed to form the adhesive patch 126.

In one example, with reference to FIG. 1, with the adhesive patch 126 formed as discussed with regard to FIG. 4, the hub 122 formed and coupled to the tube 110, the hub 122 is coupled to the first backing layer 200. In one example, the hub 122 is coupled to the first backing layer 200 using ultrasonic welding. It should be noted that other techniques may be employed, if desired, to couple the hub 122 to the adhesive patch 126. For example, additional adhesives, including, but not limited to ultraviolet light cured adhesives, heat-glued adhesives, etc., may be employed to couple the hub 122 to the adhesive patch 126. With the hub 122 coupled to the adhesive patch 126, the infusion set 104, including the infusion unit 112, may be distributed to a user. Once received by a user, the user may couple the infusion set 104 to the fluid infusion device 102 via the connector assembly 114 to establish fluid communication between the fluid infusion device 102 and the infusion unit 112. Then, the infusion unit 112 is coupled to the body B of the user. For example, the liner 208 of the adhesive patch 126 is removed to expose the second adhesive layer 206. With reference to FIG. 2, the infusion unit 112 is coupled or inserted onto the body B of the user with an insertion device, so that at least a portion of the fluid outlet 128 (FIG. 1) extends into the body B of the user and the second adhesive layer 206 of the adhesive patch 126 is coupled to the body B of the user. With the infusion unit 112 coupled to the user, fluid from the fluid reservoir of the fluid infusion device 102 flows through the infusion unit 112 into the body B of the user and the adhesive patch 126, including the first backing layer 200, the first adhesive layer 202, the second backing layer 204 and the second adhesive layer 206, provides breathability and environmental factor resistance to ensure comfort during the extended wear of the adhesive patch 126.

For example, during a trial study, the adhesive patch 126 maintained the infusion unit 112 coupled to the body B (FIG. 2) for multiple users up to 8 days. During the use of the adhesive patch 126, the users engaged in water-based activities, such as baths, showers, etc., without the adhesive patch 126 becoming uncoupled from the body B (FIG. 2) of the users for at least 8 days. The adhesive patch 126 also withstood mechanical pulling on the adhesive patch 126 and/or infusion unit 112 for up to 8 days without uncoupling from the body B of the users. Further, the adhesive patch 126 had reduced peeling of edges of the adhesive patch 126 off the body B of the user. Generally, of the adhesive patches 126 worn by the multiple users, in sum, the adhesive patches 126 had a survival rate of greater than about 95% for 7 days. In addition, while being worn for the extended period of time by the users, the users reported that the adhesive patch 126 maintained an acceptable overall appearance; that the amount of time the particular user was aware of the adhesive patch 126 during wear was acceptable; the level of skin irritation experienced by the adhesive patch 126 was substantially none; the process of cleaning the skin (to remove remaining adhesive from the adhesive patch 126, if any) was acceptable; and that the level of discomfort experienced during removal of the adhesive patch 126 from the skin was substantially none at all.

Thus, the adhesive patch 126 provides improved longevity (up to 8 days of wear), without impacting user comfort. Moreover, the adhesive patch 126 reduces a peeling of the edges of the adhesive patch 126, which further improves the longevity of the adhesive patch 126. The use of the second backing layer 204 provides environmental factor resistance, such as resistance to water, while the first backing layer 200 improves manufacturability of the medical device, such as the infusion unit 112, by enabling the hub 122 to be ultrasonically bonded to the first backing layer 200. Further, the increased longevity of the adhesive patch 126, in turn, results in an increased longevity or wear life for the infusion unit 112, which improves user satisfaction by reducing a number of replacements of the infusion unit 112.

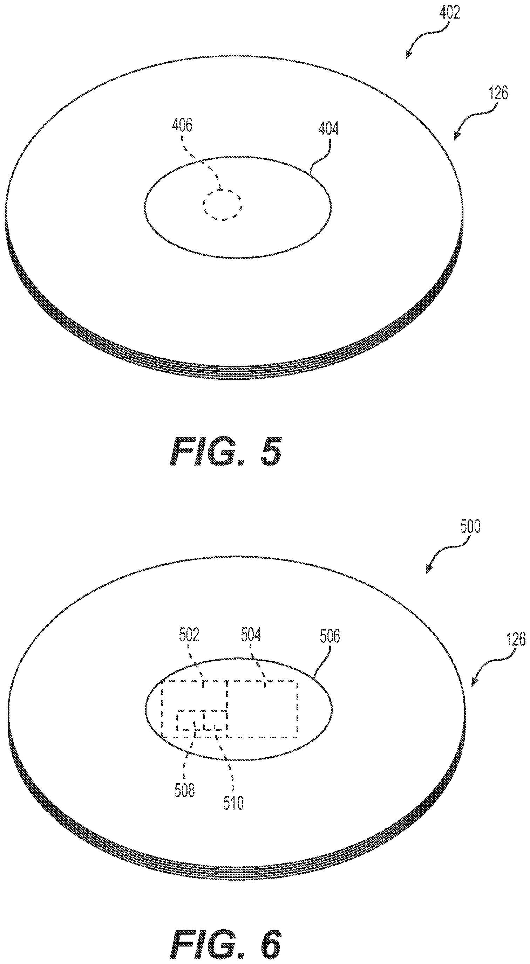

It should be noted that in other embodiments, the adhesive patch 126 may used with other medical devices that may be coupled to a body of a user to extend the wear life of the associated medical device, such as a physiological characteristic sensor. For example, with reference to FIG. 5, a medical device or physiological characteristic sensor 402 is shown. In one example, the physiological characteristic sensor 402 is a glucose sensor, such as a continuous glucose sensor of the type used by diabetic users. For the sake of brevity, conventional aspects and technology related to glucose sensors and glucose sensor fabrication may not be described in detail here. In this regard, known and/or conventional aspects of glucose sensors and their manufacturing may be of the type described in, but not limited to: United States patent numbers 6,892,085, 7,468,033 and 9,295,786; and United States patent application number 2009/0299301. In this example, the physiological characteristic sensor 402 includes a hub or sensor base 404 and a glucose sensor 406 that is coupled to the sensor base 404. The sensor base 404 gives structural support to the glucose sensor 406, and facilitates entry of the glucose sensor 406 into the body of the user. The sensor base 404 may also feature electrical and physical interfaces and elements that accommodate the sensor electronics module (not shown), such as the wireless transmitter that communicates with the infusion pump, the monitor device, or the like. In certain embodiments, the physiological characteristic sensor 402 may include the monitor device coupled to the sensor base 404 such that the physiological characteristic sensor 402 is a continuous glucose monitor. The glucose sensor 406 is an electrochemical sensor that includes the glucose oxidase enzyme, as is well understood by those familiar with glucose sensor technology. The glucose oxidase enzyme enables the glucose sensor 406 to monitor blood glucose levels in a diabetic patient or user by effecting a reaction of glucose and oxygen.

The physiological characteristic sensor 402 also includes the adhesive patch 126, which in this example, includes the includes the first backing layer 200, the first adhesive layer 202, the second backing layer 204, the second adhesive layer 206 and the liner 208. With the liner 208 removed, the adhesive patch 126 couples the physiological characteristic sensor 402 to the body of the user, and provides increased longevity without compromising user comfort.

It should be noted that in other embodiments, the adhesive patch 126 may used with other medical devices that may be coupled to a body of a user to extend the wear life of the associated medical device, such as a fluid infusion system. For example, with reference to FIG. 6, a medical device or fluid infusion system 500 is shown. In one example, the fluid infusion system 500 includes a fluid infusion device 502 (e.g., an insulin patch pump) and an infusion unit 504, which is disposed within and fluidly coupled to the fluid infusion device 502. The fluid infusion device 502 and the infusion unit 504 are each coupled to a hub 506, and the hub 506 is coupled to the body of the user via the adhesive patch 126. In this example, the fluid infusion device 502 accommodates an internal fluid reservoir 508 for the fluid to be delivered to the user. A tube 510 represents the fluid flow path that couples the fluid reservoir 508 to the infusion unit 504. When installed as depicted in FIG. 6, the tube 510 extends internally from the fluid reservoir 508 to the internal infusion unit 504, which in turn provides a fluid pathway to the body of the user through a cannula.

The fluid infusion system 500 includes the adhesive patch 126, which in this example, includes the first backing layer 200, the first adhesive layer 202, the second backing layer 204, the second adhesive layer 206 and the liner 208. With the liner 208 removed, the adhesive patch 126 couples the fluid infusion system 500 to the body of the user, and provides increased longevity without compromising user comfort.

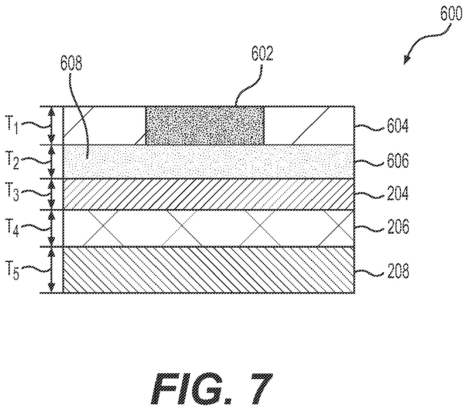

It should be noted that in still other embodiments, the adhesive patch 126 may be configured differently to provided extended wear for a medical device. For example, with reference to FIG. 7, a cross-section of an adhesive patch 600 is shown. The adhesive patch 600 may be used with a medical device, including, but not limited to the infusion unit 112, the physiological characteristic sensor 402 (FIG. 5), the fluid infusion system 500 (FIG. 6), etc. In addition, the adhesive patch 600 may be a stand-alone device, if desired. As the adhesive patch 600 includes the same or similar components as the adhesive patch 126 discussed with regard to FIGS. 1-6, the same reference numerals will be used to denote the same or similar components. In the example of FIG. 7, the adhesive patch 600 includes a control system 602, a first backing layer 604, a medicament layer 606, the second backing layer 204, the second adhesive layer 206 and the liner 208. Generally, the first backing layer 604 enables the coupling of the adhesive patch 600 to the hub 122 (FIG. 2), the hub or sensor base 404 (FIG. 5) or the hub 506 (FIG. 6) with more efficient manufacturing techniques, such as ultrasonic welding, while the second backing layer 204 provides flexibility, environmental factor resistance, such as water resistance, and breathability that provides user comfort. The combination of the first backing layer 604 with the second backing layer 204, thus, provides the adhesive patch 126 with longevity, environmental factor resistance, user comfort and ease of manufacturing. It should be noted that the adhesive patch 600 may have any desired shape for the particular medical device, including, but not limited to non-circular, trilobular, etc.

The first backing layer 604 couples the adhesive patch 600 to the hub 122 (FIG. 2), the hub or sensor base 404 (FIG. 5) or the hub 506 (FIG. 6). In one example, the first backing layer 604 is composed of a thermoplastic woven or non-woven material, including, but not limited to polycarbonate, polycarbonate urethane, polyamide, nylon, polypropylene, polyethylene, cellulose acetate, polyacrylate, polyester, etc. In one example, the first backing layer 604 is composed of a thermoplastic woven or non-woven material that is suitable for ultrasonic welding to the hub 122 (FIG. 2), the hub or sensor base 404 (FIG. 5) or the hub 506 (FIG. 6). In this non-limiting example, the first backing layer 604 is composed of polyester. The first backing layer 604 may also be composed of a thermoplastic woven or non-woven material that has similar molecular structures as the hub 122 (FIG. 2), the hub or sensor base 404 (FIG. 5) or the hub 506 (FIG. 6). Generally, the first backing layer 604 provides ease of manufacturing of the medical device, such as the infusion unit 112 (FIG. 2), the physiological characteristic sensor 402 (FIG. 5), the fluid infusion system 500 (FIG. 6), as the first backing layer 604 is able to be bonded to the hub 122 (FIG. 2), the hub or sensor base 404 (FIG. 5) or the hub 506 (FIG. 6) via ultrasonic welding. The first backing layer 604 has the thickness T1 of between about 3.0 mils to about 15 mils, and in one example, the thickness T1 is between about 8.0 mils to about 10 mils. The first backing layer 604 is physically coupled to or adhered to the medicament layer 606.

The control system 602 is coupled to the first backing layer 604. In this embodiment, the control system 602 is integrally formed with the first backing layer 604, however, in other embodiments, the control system 602 may be coupled to a top surface of the first backing layer 604. Generally, the control system 602 is coupled to the first backing layer 604 so as to be spaced apart from the respective one of the hub 122 (FIG. 2), the hub or sensor base 404 (FIG. 5) or the hub 506 (FIG. 6). By being spaced apart from the hub 122 (FIG. 2), the hub or sensor base 404 (FIG. 5) or the hub 506 (FIG. 6), the control system 602 is independently accessible by the user. In other embodiments, however, the control system 602 may be integral with, disposed within or part of the hub 122 (FIG. 2), the hub or sensor base 404 (FIG. 5) or the hub 506 (FIG. 6). Generally, the control system 602 is coupled to the first backing layer 604 so as to be in communication with the medicament layer 606 to enable the release of the medicament to the user.

The control system 602 is responsive to user input to dispense a medicament 608 contained within the medicament layer 606. In one example, the control system 602 comprises a heating coil, which is responsive to an input to heat the medicament layer 606 to release the medicament 608. In other examples, the control system 602 comprises a humidifier, which is capable of creating moisture, which causes the release of the medicament 608 from the medicament layer 606. In other embodiments, the control system 602 is a mechanical device, such as a button, which is manipulatable by the user to apply pressure to release the medicament 608 from the medicament layer 606. As yet another example, the control system 602 may include a receiver that is in communication with a portable device associated with the user, and a processor that is responsive to a signal received by the receiver to generate an impulse, such as an electrical impulse, vibratory impulse, etc. to release the medicament 608 from the medicament layer 606. Thus, in certain embodiments, the control system 602 may include a processor, memory and a source of power, that receives a transmitted user input and generates an impulse to dispense the medicament 608 from the medicament layer 606. Moreover, in certain embodiments, the control system 602 may be external to the adhesive patch 600. For example, an external heat source and/or pressure source may be applied to the adhesive patch 600 to release the medicament 608 from the medicament layer 606.

The medicament layer 606 couples the first backing layer 604 to the second backing layer 204 and includes the medicament 608. Stated another way, the medicament layer 606 is sandwiched between, and is adhesively coupled to the first backing layer 604 and the second backing layer 204. Thus, in this example, the medicament layer 606 comprises the first adhesive layer. The medicament layer 606 is spaced apart from or not in direct contact with the body B (FIG. 2) or skin of the user, and the medicament 608 disposed within the medicament layer 606 diffuses into the skin or body B of the user through the second backing layer 204 and the second adhesive layer 206. By providing the medicament layer 606 between the first backing layer 604 and the second backing layer 204, the medicament layer 606 may provide for a long term release of the medicament 608 over the use of the adhesive patch 600.

The medicament layer 606 may be coated onto or penetrated into the first backing layer 604. Thus, the medicament layer 606 and the first backing layer 604 may be separately or integrally formed. In one example, the medicament layer 606 is composed of a polyacrylic, bio-based acrylate, silicone adhesive, ethylene-vinyl-acetate (EVA) or an adhesive material that bonds securely to the second backing layer 204. The medicament 608 is contained within or encapsulated by the medicament layer 606 such that the medicament 608 is released from the medicament layer 606 based on the receipt of the input from the control system 602. The medicament 608 comprises any suitable medicine for use by the user, including, but not limited to, anti-inflammatory medicine, anti-microbial medicine, pain-relieving medicine, pain-killing medicine, nitroglycerin, scopolamine, clonidine, rivastigmine, drugs to treat pulmonary hypertension, iron chelation drugs, anti-cancer treatments, medications, vitamins, hormones, etc. The medicament layer 606 has the thickness T2 of between about 1.0 mils to about 5.0 mils, and in one example, the thickness T2 is between about 2.0 mils to about 4.0 mils.

The second backing layer 204 is coupled between the medicament layer 606 and the second adhesive layer 206. The second backing layer 204 is positioned between the medicament layer 606 and the second adhesive layer 206. In certain instances, when the second adhesive layer 206 (FIG. 2) penetrates through the second backing layer 204 or when under pressure, the second backing layer 204 may contact the skin of the user. The second adhesive layer 206 couples the second backing layer 204 to the liner 208. Stated another way, the second adhesive layer 206 is sandwiched between, and is adhesively coupled to the second backing layer 204 and the liner 208. The second adhesive layer 206 may be coated onto or penetrated into the second backing layer 204. The liner 208 is removably coupled to the second adhesive layer 206, such that when the liner 208 is removed, the second adhesive layer 206 may be placed in direct contact with the body B (FIG. 2) or skin of the user. Thus, in this example, the second adhesive layer 206 couples the adhesive patch 600 to the body B (FIG. 2) of the user.

As the adhesive patch 600 may be assembled similarly to the assembly of the adhesive patch 126 discussed with regard to FIG. 4, the assembly of the adhesive patch 600 will not be discussed in great detail herein. Briefly, the first backing layer 604, the control system 602 and the medicament layer 606 are provided as a first subassembly; and the second backing layer 204, the second adhesive layer 206 and the liner 208 are provided as a second subassembly. The medicament layer 606 may be coated on or penetrated into the first backing layer 604 and the control system 602 may be coupled to the first backing layer 604 in an initial processing step to form the first subassembly. The medicament 608 may be pre-loaded into the medicament layer 606 or may be spray coated onto the medicament layer 606 in an initial processing step. The second adhesive layer 206 may be coated on or penetrated into the second backing layer 204 in an initial processing step, and the liner 208 may be adhered to the second adhesive layer 206 to form the second subassembly. A laminator, such as the laminator 304 of FIG. 4, may heat and soften or melt at least the medicament layer 606 to couple or adhere the medicament layer 606 to the second backing layer 204 to form the adhesive patch 600. The adhesive patch 600 may be cut or stamped in a post processing step to arrive at the desired size and shape for use with the particular medical device. It should be noted that the use of the laminator 304 of FIG. 4 is merely an example, as pressure, light or ultrasonic welding may be used couple the medicament layer 606 to the second backing layer 204. In addition, it should be noted that the method of assembling the adhesive patch 600 is merely exemplary as any suitable approach may be employed to form the adhesive patch 600.

In one example, with the adhesive patch 600 formed, the adhesive patch 600 is coupled to the respective one of the hub 122 (FIG. 2), the hub or sensor base 404 (FIG. 5) or the hub 506 (FIG. 6) via ultrasonic welding, for example. Once the adhesive patch 600 is coupled to the body of the user, the user may activate the control system 602, via pressing for example, to initiate the release of the medicament 608 as needed. The medicament 608 is released by diffusing through the second backing layer 204 and the second adhesive layer 206 to enter through the skin of the patient. Thus, the adhesive patch 600 provides increased longevity without compromising user comfort, while also enabling the user to receive the medicament 608. In the example of the medicament 608 as an anti-inflammatory or anti-microbial medicament, the wear of the adhesive patch 600 may be extended for greater than 8 days with reduced risk.

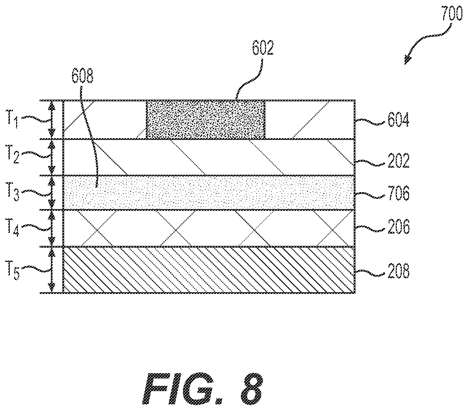

It should be noted that in still other embodiments, the adhesive patch 126 may be configured differently to provided extended wear for a medical device. For example, with reference to FIG. 8, a cross-section of an adhesive patch 700 is shown. The adhesive patch 700 may be used with a medical device, including, but not limited to the infusion unit 112, the physiological characteristic sensor 402 (FIG. 5), the fluid infusion system 500 (FIG. 6), etc. In addition, the adhesive patch 700 may be a stand-alone device, if desired. As the adhesive patch 700 includes the same or similar components as the adhesive patch 126 discussed with regard to FIGS. 1-6 and the adhesive patch 600 discussed with regard to FIG. 7, the same reference numerals will be used to denote the same or similar components. In the example of FIG. 8, the adhesive patch 700 includes the control system 602, the first backing layer 604, the first adhesive layer 202, a medicament layer 706, the second adhesive layer 206 and the liner 208. Generally, the first backing layer 604 enables the coupling of the adhesive patch 600 to the hub 122 (FIG. 2), the hub or sensor base 404 (FIG. 5) or the hub 506 (FIG. 6) with more efficient manufacturing techniques, such as ultrasonic welding. It should be noted that the adhesive patch 700 may have any desired shape for the particular medical device, including, but not limited to non-circular, trilobular, etc. As discussed, the first backing layer 604 is composed of a thermoplastic woven or non-woven material, including, but not limited to polycarbonate, polycarbonate urethane, polyamide, nylon, polypropylene, polyethylene, cellulose acetate, polyacrylate, polyester, etc. The first backing layer 604 is physically coupled to or adhered to the first adhesive layer 202.

The control system 602 is coupled to the first backing layer 604. In this embodiment, the control system 602 is integrally formed with the first backing layer 604, however, in other embodiments, the control system 602 may be coupled to a top surface of the first backing layer 604. Generally, the control system 602 is coupled to the first backing layer 604 so as to be in communication with the medicament layer 706 to enable the release of the medicament to the user.

The first adhesive layer 202 couples the first backing layer 604 to the medicament layer 706. Stated another way, the first adhesive layer 202 is sandwiched between, and is adhesively coupled to the first backing layer 604 and the medicament layer 706. Thus, the first adhesive layer 202 is spaced apart from or not in direct contact with the body B (FIG. 2) or skin of the user. The first adhesive layer 202 may be coated onto or penetrated into the first backing layer 604. Thus, the first adhesive layer 202 and the first backing layer 604 may be separately or integrally formed. In one example, the first adhesive layer 202 is composed of a polyacrylic, silicone adhesive, or an adhesive material that bonds securely to the medicament layer 706.

The medicament layer 706 is coupled between the first adhesive layer 202 and the second adhesive layer 206 and includes the medicament 608. In this example, the medicament layer 706 comprises the second backing layer. The medicament layer 706 is spaced apart from the body B (FIG. 2) or skin of the user and the medicament 608 disposed within the medicament layer 706 diffuses into the skin or body B of the user through the second adhesive layer 206. In certain instances, when the second adhesive layer 206 penetrates through the medicament layer 706 or when under pressure, the medicament layer 706 may contact the skin of the user. In one example, the medicament layer 706 is composed of a stretchable, breathable and water-resistant polymeric material, including, but not limited to polyethylene, polypropylene, silicone film/sheet, acrylic elastomer, polyurethane, etc. that is capable of retaining the medicament 608. In this non-limiting example, the medicament layer 706 is composed of a polyurethane, and the medicament 608 is contained or encapsulated within the polyurethane. Generally, the medicament layer 706 provides environmental factor resistance, such as water resistance, while maintaining breathability, flexibility and providing the medicament 608, which provides the user with comfort and treatment during the extended use of the adhesive patch 126. The medicament layer 706 has the thickness T3 of between about 3.0 mils to about 9.0 mils, and in one example, the thickness T3 is between about 5.0 mils to about 7.0 mils. Thus, generally, the second backing layer 204 has the thickness T3, which is less than the thickness T1 of the first backing layer 200. The medicament layer 706 is coupled to or adhered to the second adhesive layer 206. By providing the medicament layer 706 between the first backing layer 604 and the second adhesive layer 206, the medicament layer 706 may provide for an intermediate term of release of the medicament 608 over the use of the adhesive patch 700, which is less than the term or period of release provided by the medicament layer 606 of the adhesive patch 600.

Generally, as discussed, the medicament layer 706 is responsive to the control system 602 to dispense the medicament 608 onto the body of the user. As discussed with regard to FIG. 6, the medicament 608 comprises any suitable medicine for use by the user, including, but not limited to, anti-inflammatory medicine, anti-microbial medicine, pain-relieving medicine, pain-killing medicine, nitroglycerin, scopolamine, clonidine, rivastigmine, drugs to treat pulmonary hypertension, iron chelation drugs, anti-cancer treatments, medications, vitamins, hormones, etc.

The second adhesive layer 206 couples the medicament layer 706 to the liner 208. Stated another way, the second adhesive layer 206 is sandwiched between, and is adhesively coupled to the medicament layer 706 and the liner 208. The second adhesive layer 206 may be coated onto or penetrated into the medicament layer 706. The liner 208 is removably coupled to the second adhesive layer 206, such that when the liner 208 is removed, the second adhesive layer 206 may be placed in direct contact with the body B (FIG. 2) or skin of the user. Thus, in this example, the second adhesive layer 206 couples the adhesive patch 700 to the body B (FIG. 2) of the user.

As the adhesive patch 700 may be assembled similarly to the assembly of the adhesive patch 126 discussed with regard to FIG. 4 and the adhesive patch 600, the assembly of the adhesive patch 700 will not be discussed in great detail herein. Briefly, the first backing layer 604, the control system 602 and the first adhesive layer 202 are provided as a first subassembly; and the medicament layer 706, the second adhesive layer 206 and the liner 208 are provided as a second subassembly. The first adhesive layer 202 may be coated on or penetrated into the first backing layer 604 and the control system 602 may be coupled to the first backing layer 604 in an initial processing step to form the first subassembly. The second adhesive layer 206 may be coated on or penetrated into the medicament layer 706 in an initial processing step, and the liner 208 may be adhered to the second adhesive layer 206 to form the second subassembly. The medicament 608 may be pre-loaded into the medicament layer 706 or may be spray coated onto the medicament layer 706 in an initial processing step. A laminator, such as the laminator 304 of FIG. 4, may heat and soften or melt at least the first adhesive layer 202 to couple or adhere the medicament layer 706 to the first adhesive layer 202 to form the adhesive patch 700. The adhesive patch 700 may be cut or stamped in a post processing step to arrive at the desired size and shape for use with the particular medical device. It should be noted that the use of the laminator 304 of FIG. 4 is merely an example, as pressure, light or ultrasonic welding may be used couple the medicament layer 706 to the first adhesive layer 202. In addition, it should be noted that the method of assembling the adhesive patch 700 is merely exemplary as any suitable approach may be employed to form the adhesive patch 700.

In one example, with the adhesive patch 700 formed, the adhesive patch 700 is coupled to the respective one of the hub 122 (FIG. 2), the hub or sensor base 404 (FIG. 5) or the hub 506 (FIG. 6) via ultrasonic welding, for example. Once the adhesive patch 700 is coupled to the body of the user, the user may activate the control system 602, via pressing for example, to initiate the release of the medicament 608 as needed. The medicament 608 is released by diffusing through the second adhesive layer 206 to enter through the skin of the patient. Thus, the adhesive patch 700 provides increased longevity without compromising user comfort, while also enabling the user to receive the medicament 608. In the example of the medicament 608 as an anti-inflammatory or anti-microbial medicament, the wear of the adhesive patch 700 may be extended for greater than 8 days with reduced risk.

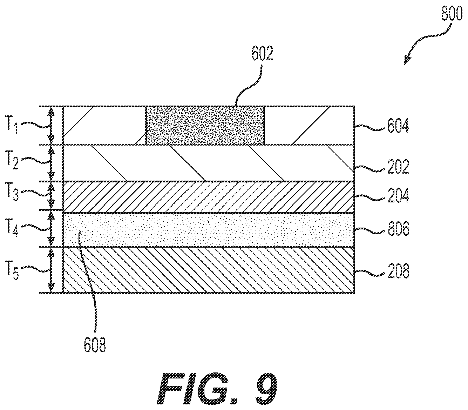

It should be noted that in still other embodiments, the adhesive patch 126 may be configured differently to provided extended wear for a medical device. For example, with reference to FIG. 9, a cross-section of an adhesive patch 800 is shown. The adhesive patch 800 may be used with a medical device, including, but not limited to the infusion unit 112, the physiological characteristic sensor 402 (FIG. 5), the fluid infusion system 500 (FIG. 6), etc. In addition, the adhesive patch 800 may be a stand-alone device, if desired. As the adhesive patch 800 includes the same or similar components as the adhesive patch 126 discussed with regard to FIGS. 1-6 and the adhesive patch 600 discussed with regard to FIG. 7, the same reference numerals will be used to denote the same or similar components. In the example of FIG. 9, the adhesive patch 800 includes the control system 602, the first backing layer 604, the first adhesive layer 202, the second backing layer 204, a medicament layer 806 and the liner 208. Generally, the first backing layer 604 enables the coupling of the adhesive patch 600 to the hub 122 (FIG. 2), the hub or sensor base 404 (FIG. 5) or the hub 506 (FIG. 6) with more efficient manufacturing techniques, such as ultrasonic welding. It should be noted that the adhesive patch 800 may have any desired shape for the particular medical device, including, but not limited to non-circular, trilobular, etc. As discussed, the first backing layer 604 is composed of a thermoplastic woven or non-woven material, including, but not limited to polycarbonate, polycarbonate urethane, polyamide, nylon, polypropylene, polyethylene, cellulose acetate, polyacrylate, polyester, etc. The first backing layer 604 is physically coupled to or adhered to the first adhesive layer 202.

The control system 602 is coupled to the first backing layer 604. In this embodiment, the control system 602 is integrally formed with the first backing layer 604, however, in other embodiments, the control system 602 may be coupled to a top surface of the first backing layer 604. Generally, the control system 602 is coupled to the first backing layer 604 so as to be in communication with the medicament layer 806 to enable the release of the medicament to the user.

The first adhesive layer 202 couples the first backing layer 604 to the second backing layer 204. Stated another way, the first adhesive layer 202 is sandwiched between, and is adhesively coupled to the first backing layer 604 and the second backing layer 204. The second backing layer 204 is coupled between the first adhesive layer 202 and the medicament layer 806. As discussed, the second backing layer 204 is composed of a stretchable, breathable and water-resistant polymeric material, including, but not limited to polyethylene, polypropylene, silicone film/sheet, acrylic elastomer, polyurethane, etc. The second backing layer 204 is coupled to or adhered to the medicament layer 806.

The medicament layer 806 is coupled between the second backing layer 204 and the liner 208, and includes the medicament 608. Stated another way, the medicament layer 806 is sandwiched between, and is adhesively coupled to the second backing layer 204 and the liner 208. In this example, the medicament layer 806 comprises the second adhesive layer. The medicament layer 806 may be coated onto or penetrated into the second backing layer 204. Thus, the medicament layer 806 and the second backing layer 204 may be separately or integrally formed. As will be discussed, the liner 208 is removably coupled to the medicament layer 806, such that when the liner 208 is removed, the medicament layer 806 may be placed in direct contact with the body B (FIG. 2) or skin of the user. Thus, in this example, the medicament layer 806 couples the adhesive patch 800 to the body B (FIG. 2) of the user. In one example, the medicament layer 806 is composed of a biocompatible adhesive, including, but not limited to, polyacrylics, bio-based acrylate, ethylene-vinyl-acetate (EVA), and silicone adhesive, and the medicament 608 is contained or encapsulated within the biocompatible adhesive. The medicament layer 806 has the thickness T4 of between about 1.0 mils to about 5.0 mils, and in one example, the thickness T4 is between about 2.0 mils to about 4.0 mils. By providing the medicament layer 806 between the second backing layer 204 and the liner 208, the medicament layer 806 may provide for a short term release of the medicament 608 over the use of the adhesive patch 800, which is less than the term or period of release provided by the medicament layer 706 of the adhesive patch 700.

Generally, as discussed, the medicament layer 806 is responsive to the control system 602 to dispense the medicament 608 onto the body of the user. As discussed with regard to FIG. 6, the medicament 608 comprises any suitable medicine for use by the user, including, but not limited to, anti-inflammatory medicine, anti-microbial medicine, pain-relieving medicine, pain-killing medicine, nitroglycerin, scopolamine, clonidine, rivastigmine, drugs to treat pulmonary hypertension, iron chelation drugs, anti-cancer treatments, medications, vitamins, hormones, etc.

The liner 208 is removably coupled to the medicament layer 806, such that when the liner 208 is removed, the medicament layer 806 may be placed in direct contact with the body B (FIG. 2) or skin of the user. Thus, in this example, the medicament layer 806 couples the adhesive patch 800 to the body B (FIG. 2) of the user.

As the adhesive patch 800 may be assembled similarly to the assembly of the adhesive patch 126 discussed with regard to FIG. 4, the adhesive patch 600 and the adhesive patch 700, the assembly of the adhesive patch 800 will not be discussed in great detail herein. Briefly, the first backing layer 604, the control system 602 and the first adhesive layer 202 are provided as a first subassembly; and the second backing layer 204, the medicament layer 806 and the liner 208 are provided as a second subassembly. The first adhesive layer 202 may be coated on or penetrated into the first backing layer 604 and the control system 602 may be coupled to the first backing layer 604 in an initial processing step to form the first subassembly. The medicament layer 806 may be coated on or penetrated into the second backing layer 204 in an initial processing step, and the liner 208 may be adhered to the medicament layer 806 to form the second subassembly. The medicament 608 may be pre-loaded into the medicament layer 806 or may be spray coated onto the medicament layer 806 in an initial processing step. A laminator, such as the laminator 304 of FIG. 4, may heat and soften or melt at least the first adhesive layer 202 to couple or adhere the second backing layer 204 to the first adhesive layer 202 to form the adhesive patch 800. The adhesive patch 800 may be cut or stamped in a post processing step to arrive at the desired size and shape for use with the particular medical device. It should be noted that the use of the laminator 304 of FIG. 4 is merely an example, as pressure, light or ultrasonic welding may be used couple the second backing layer 204 to the first adhesive layer 202. In addition, it should be noted that the method of assembling the adhesive patch 800 is merely exemplary as any suitable approach may be employed to form the adhesive patch 800.

In one example, with the adhesive patch 800 formed, the adhesive patch 800 is coupled to the respective one of the hub 122 (FIG. 2), the hub or sensor base 404 (FIG. 5) or the hub 506 (FIG. 6) via ultrasonic welding, for example. Once the adhesive patch 800 is coupled to the body of the user, the user may activate the control system 602, via pressing for example, to initiate the release of the medicament 608 as needed. Once released, the medicament 608 enters through the skin of the patient. Thus, the adhesive patch 800 provides increased longevity without compromising user comfort, while also enabling the user to receive the medicament 608. In the example of the medicament 608 as an anti-inflammatory or anti-microbial medicament, the wear of the adhesive patch 800 may be extended for greater than 8 days with reduced risk.

Thus, the adhesive patch 126 may be employed to provide longevity to a medical device that is coupled to a body of a user, including, but not limited to, an infusion set, a physiological characteristic sensor, a physiological characteristic sensor monitor and a fluid infusion system. Moreover, the adhesive patches 600, 700, 800 may be employed to provide longevity to a medical device that is coupled to a body of a user, including, but not limited to, an infusion set, a physiological characteristic sensor, a physiological characteristic sensor monitor and a fluid infusion system, while also providing the user with medicament as needed to treat a condition associated with the user.

While at least one exemplary embodiment has been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. An adhesive patch (126, 600, 700, 800) for a medical device, comprising:
a first backing layer (200) to be coupled to the medical device, the first backing layer (200) composed of a first material;
a first adhesive layer (202) coupled to the first backing layer (200);
a second backing layer (204) coupled to the first adhesive layer (202), the second backing layer (204) composed of a second material, the second material different than the first material; and
a biocompatible second adhesive layer (206) coupled to the second backing layer (204), the second adhesive layer (206) to be coupled to an anatomy;
**characterized in that** one of the first adhesive layer (202), the second backing layer (204) and the second adhesive layer (206) comprise a medicament, and the adhesive patch further comprises a control device (602) to release the medicament onto the anatomy.

2. A medical device, comprising:
a hub (122); and
the adhesive patch (126) of claim 1 coupled to the hub, the adhesive patch to couple the hub to an anatomy;
a liner (208) removably coupled to the second adhesive layer.

3. The adhesive patch of Claim 1 or the medical device of claim 2, wherein the control device (602) is coupled to the first backing layer (200, 604)

4. The adhesive patch of Claim 1 or the medical device of claim 2, wherein the control device (602) is integrally formed with the first backing layer (200, 604)

5. The adhesive patch of Claim 1 or the medical device of claim 2, wherein the control device (602) comprises a heating coil or a humidifier.

6. The adhesive patch of Claim 1 or the medical device of claim 2, wherein the first adhesive layer (202) is composed of a third material, the second adhesive layer (206) is composed of a fourth material, and the fourth material is different than the third material.

7. The adhesive patch of Claim 2 or the medical device of claim 2, wherein the first material, the second material, the third material and the fourth materials are each different.

8. The adhesive patch of Claim 1, further comprising a liner (208) removably coupled to the second adhesive layer.

9. The adhesive patch of Claim 1 or the medical device of claim 2, wherein the first material is selected from the group comprising polycarbonate, polycarbonate urethane, polyamide, nylon, polypropylene, polyethylene, cellulose acetate, polyacrylate and polyester.

10. The adhesive patch of Claim 1 or the medical device of claim 2, wherein the first material is polyester and the second material is polyurethane.

11. The adhesive patch of Claim 1 or the medical device of claim 2, wherein the second material is selected from the group comprising polyethylene, polypropylene, silicone film, silicone sheet, acrylic elastomer and polyurethane.

12. The medical device of Claim 2, wherein the medical device is an infusion unit, the hub defines a fluid flow path to receive a fluid and the infusion unit is fluidly coupled to a fluid infusion device to receive the fluid.

13. The medical device of Claim 2, wherein the medical device is a physiological characteristic sensor to be coupled to the anatomy to observe a physiological characteristic.

14. The medical device of Claim 2, wherein the medical device is a fluid infusion system to be coupled to the anatomy to define a fluid flow path into the anatomy.

15. The fluid infusion system of claim 14 wherein:
the first backing layer (200) is coupled to the hub (122) via ultrasonic welding, the first backing layer (200) composed of a first material that facilitates the ultrasonic welding of the first backing layer to the hub; and
the second backing layer (204) is flexible and water resistant.

## Patentansprüche

1. Klebepflaster (126, 600, 700, 800) für eine medizinische Vorrichtung, umfassend:
eine erste Trägerschicht (200), die mit der medizinischen Vorrichtung gekoppelt werden soll, wobei die erste Trägerschicht (200) aus einem ersten Material besteht;
eine erste Klebeschicht (202), die mit der ersten Trägerschicht (200) gekoppelt ist;
eine zweite Trägerschicht (204), die mit der ersten Klebeschicht (202) gekoppelt ist, wobei die zweite Trägerschicht (204) aus einem zweiten Material besteht, wobei sich das zweite Material von dem ersten Material unterscheidet; und
eine biokompatible zweite Klebeschicht (206), die mit der zweiten Trägerschicht (204) gekoppelt ist, wobei die zweite Klebeschicht (206) mit einer Anatomie gekoppelt ist;
**dadurch gekennzeichnet, dass**
eine von der ersten Klebeschicht (202), der zweiten Trägerschicht (204) und der zweiten Klebeschicht (206) ein Arzneimittel umfasst und das Klebepflaster ferner eine Steuervorrichtung (602) umfasst, um das Arzneimittel auf die Anatomie zu lösen.

2. Medizinische Vorrichtung, umfassend:
eine Nabe (122); und
das Klebepflaster (126) nach Anspruch 1, das mit der Nabe gekoppelt ist, wobei das Klebepflaster die Nabe mit einer Anatomie koppelt;
eine Schutzfolie (208), die mit der zweiten Klebeschicht entfernbar gekoppelt ist.

3. Klebepflaster nach Anspruch 1 oder medizinische Vorrichtung nach Anspruch 2, wobei die Steuervorrichtung (602) mit der ersten Trägerschicht (200, 604) gekoppelt ist

4. Klebepflaster nach Anspruch 1 oder medizinische Vorrichtung nach Anspruch 2, wobei die Steuervorrichtung (602) mit der ersten Trägerschicht (200, 604) einstückig ausgebildet ist

5. Klebepflaster nach Anspruch 1 oder medizinische Vorrichtung nach Anspruch 2, wobei die Steuervorrichtung (602) eine Heizspule oder einen Befeuchter umfasst.

6. Klebepflaster nach Anspruch 1 oder medizinische Vorrichtung nach Anspruch 2, wobei die erste Klebeschicht (202) aus einem dritten Material besteht, wobei die zweite Klebeschicht (206) aus einem vierten Material besteht und das vierte Material sich von dem dritten Material unterscheidet.

7. Klebepflaster nach Anspruch 2 oder medizinische Vorrichtung nach Anspruch 2, wobei das erste Material, das zweite Material, das dritte Material und die vierten Materialien jeweils unterschiedlich sind.

8. Klebepflaster nach Anspruch 1, ferner umfassend eine Schutzfolie (208), die mit der zweiten Klebeschicht entfernbar gekoppelt ist.

9. Klebepflaster nach Anspruch 1 oder medizinische Vorrichtung nach Anspruch 2, wobei das erste Material aus der Gruppe ausgewählt ist, umfassend Polycarbonat, Polycarbonaturethan, Polyamid, Nylon, Polypropylen, Polyethylen, Celluloseacetat, Polyacrylat und Polyester.

10. Klebepflaster nach Anspruch 1 oder medizinische Vorrichtung nach Anspruch 2, wobei das erste Material Polyester ist und das zweite Material Polyurethan ist.

11. Klebepflaster nach Anspruch 1 oder medizinische Vorrichtung nach Anspruch 2, wobei das zweite Material aus der Gruppe ausgewählt ist, umfassend Polyethylen, Polypropylen, Siliconfilm, Siliconfolie, Acrylelastomer und Polyurethan.

12. Medizinische Vorrichtung nach Anspruch 2, wobei die medizinische Vorrichtung eine Infusionseinheit ist, wobei die Nabe einen Fluidströmungsweg definiert, um ein Fluid aufzunehmen, und die Infusionseinheit mit einer Fluidinfusionsvorrichtung fluidisch gekoppelt ist, um das Fluid aufzunehmen.

13. Medizinische Vorrichtung nach Anspruch 2, wobei die medizinische Vorrichtung ein Sensor für physiologische Eigenschaften ist, der mit der Anatomie gekoppelt werden soll, um eine physiologische Eigenschaft zu beobachten.

14. Medizinische Vorrichtung nach Anspruch 2, wobei die medizinische Vorrichtung ein Fluidinfusionssystem ist, das mit der Anatomie gekoppelt werden soll, um einen Fluidströmungsweg in die Anatomie zu definieren.

15. Fluidinfusionssystem nach Anspruch 14, wobei:
die erste Trägerschicht (200) über Ultraschallschweißen mit der Nabe (122) gekoppelt ist, wobei die erste Trägerschicht (200) aus einem ersten Material besteht, das Ultraschallschweißen der ersten Trägerschicht mit der Nabe erleichtert; und
die zweite Trägerschicht (204) flexibel und wasserbeständig ist.

## Revendications

1. Timbre adhésif (126, 600, 700, 800) pour un dispositif médical, comprenant :
une première couche de support (200) destinée à être accouplée au dispositif médical, la première couche de support (200) étant composée d'un premier matériau ;
une première couche adhésive (202) accouplée à la première couche de support (200) ;
une seconde couche de support (204) accouplée à la première couche adhésive (202), la seconde couche de support (204) composée d'un deuxième matériau, le deuxième matériau différent du premier matériau ; et
une seconde couche adhésive biocompatible (206) accouplée à la seconde couche de support (204), la seconde couche adhésive (206) destinée à être accouplée à une anatomie ;
**caractérisé en ce que**
l'une parmi la première couche adhésive (202), la seconde couche de support (204), et la seconde couche adhésive (206) comprend un médicament, et le timbre adhésif comprend en outre un dispositif de commande (602) pour libérer le médicament sur l'anatomie.

2. Dispositif médical, comprenant :
un moyeu (122) ; et
un timbre adhésif (126) selon la revendication 1 accouplée au moyeu, le timbre adhésif pour coupler le moyeu à une anatomie ;
un revêtement (208) accouplé de manière amovible à la seconde couche adhésive.

3. Timbre adhésif selon la revendication 1 ou dispositif médical selon la revendication 2, dans lequel le dispositif de commande (602) est accouplé à la première couche de support (200, 604)

4. Timbre adhésif selon la revendication 1 ou dispositif médical selon la revendication 2, dans lequel le dispositif de commande (602) est formé d'un seul tenant avec la première couche de support (200, 604)

5. Timbre adhésif selon la revendication 1 ou dispositif médical selon la revendication 2, dans lequel le dispositif de commande (602) comprend une bobine de chauffage ou un humidificateur.

6. Timbre adhésif selon la revendication 1 ou dispositif médical selon la revendication 2, dans lequel la première couche adhésive (202) est composée d'un troisième matériau, la seconde couche adhésive (206) est composée d'un quatrième matériau, et le quatrième matériau est différent du troisième matériau.

7. Timbre adhésif selon la revendication 2 ou dispositif médical selon la revendication 2, dans lequel le premier matériau, le deuxième matériau, le troisième matériau et les quatrièmes matériaux sont chacun différents.

8. Timbre adhésif selon la revendication 1, comprenant en outre un revêtement (208) accouplé de manière amovible à la seconde couche adhésive.

9. Timbre adhésif selon la revendication 1 ou dispositif médical selon la revendication 2, dans lequel le premier matériau est choisi dans le groupe comprenant le polycarbonate, le polycarbonate uréthane, le polyamide, le nylon, le polypropylène, le polyéthylène, l'acétate de cellulose, le polyacrylate et le polyester.

10. Timbre adhésif selon la revendication 1 ou dispositif médical selon la revendication 2, dans lequel le premier matériau est du polyester et le deuxième matériau est du polyuréthane.

11. Timbre adhésif selon la revendication 1 ou dispositif médical selon la revendication 2, dans lequel le deuxième matériau est choisi dans le groupe comprenant le polyéthylène, le polypropylène, le film de silicone, la feuille de silicone, l'élastomère acrylique et le polyuréthane.

12. Dispositif médical selon la revendication 2, dans lequel le dispositif médical est une unité d'infusion, le moyeu définit un trajet d'écoulement de fluide pour recevoir un fluide et l'unité d'infusion est accouplée de manière fluidique à un dispositif d'infusion de fluide pour recevoir le fluide.

13. Dispositif médical selon la revendication 2, dans lequel le dispositif médical est un capteur de caractéristique physiologique à coupler à l'anatomie pour observer une caractéristique physiologique.

14. Dispositif médical selon la revendication 2, dans lequel le dispositif médical est un système d'infusion de fluide à coupler à l'anatomie pour définir un trajet d'écoulement de fluide dans l'anatomie.

15. Système d'infusion de fluide selon la revendication 14, dans lequel :
la première couche de support (200) est accouplée au moyeu (122) par soudage par ultrasons, la première couche de support (200) composée d'un premier matériau qui facilite le soudage par ultrasons de la première couche de support au moyeu ; et
la seconde couche de support (204) est souple et résistante à l'eau.
